# EUROPÄISCHE PATENTSCHRIFT

(11) **EP 2 231 082 B1**
(45) Veröffentlichungstag und Bekanntmachung des Hinweises auf die Patenterteilung: **18.05.2011**
(21) Anmeldenummer: 08865125.2
(22) Anmeldetag: 16.12.2008
(51) Int. Cl.: A61F 5/01

(54) **KORREKTURGELENK**
CORRECTIVE JOINT
ARTICULATION AVEC CORRECTION

(30) Priorität: 21.12.2007 DE 102007062961
(43) Veröffentlichungstag der Anmeldung: 29.09.2010
(73) Patentinhaber: Otto Bock HealthCare GmbH, 37115 Duderstadt (DE)
(72) Erfinder: SCHILLING, Matthias, 37345 Weissenborn-Lüderode (DE); JARJOUR, Wissam, 15907 Lübben (DE)
(74) Vertreter: Plöger, Jan Manfred
(86) Internationale Anmeldenummer: PCT/DE2008/002088
(87) Internationale Veröffentlichungsnummer: WO 2009/079992

(56) Entgegenhaltungen:
- EP-A- 1 579 829
- DE-A1- 19 606 092

## Beschreibung

Die Erfindung betrifft ein Gelenk gemäß Anspruch 1.

Ein derartiges Gelenk ist aus der EP 1 579 829 A bekannt. Nachteilig an diesem Gelenk ist, dass es aufwändig anzulegen ist, weil bei jedem Anlegen die Winkeleinstellung neu vorgenommen werden muss.

Ein winkelverstellbares Gelenk ist auch aus der DE 196 06 092 A1 bekannt und wird beispielsweise in Orthesen für verschiedene Körperteile eingesetzt. Die beiden Gelenkarme sind in dem Bereich, in dem sie aneinander gelagert sind, mit Löchern versehen. Bei bestimmten Werten des zwischen den beiden Gelenkarmen eingeschlossenen Spreizwinkels kommen Löcher des einen Gelenkarmes und Löcher des zweiten Gelenkarmes zur Überdeckung. In diesem Fall werden die beiden Schwenkarme durch einen durch die beiden sich überdeckenden Löcher gesteckten Stift miteinander verbunden und das Gelenk ist arretiert. Wird der Stift entfernt, sind die beiden Gelenkarme frei gegeneinander schwenkbar.

Der Stift kann auch in nur in einem Gelenkarm vorgesehene Löcher gesteckt werden. Im Bereich dieser Löcher weist der andere Gelenkarm einen geringeren Radius von der Schwenkachse auf und verfügt über eine Anschlagsnase. Diese schlägt bei einem durch den Stift festgelegten Anschlagswinkel an den Stift an. Dadurch wird der mögliche Schwenkwinkel in einer Richtung begrenzt.

Durch die Verwendung derartiger Gelenke in Orthesen für beispielsweise Knie oder Ellenbogen können die entsprechenden Körperglieder in einer bestimmten Position relativ zu einander fixiert werden. Ein Bewegen des Körpergelenks ist mit arretiertem Orthesengelenk nicht möglich. Zum Anlegen der Orthese ist es jedoch praktisch und bequemer für den Patienten, das Orthesengelenk in die gewünschte Position schwenken zu können.

Nachteilig ist, dass die Verriegelung nur in bestimmten Winkelabständen möglich ist und Zwischeneinstellungen nicht möglich sind. Zudem ist auch der Arretiervorgang selbst mühsam und unpraktisch. Im angelegten Zustand der Orthese müssen die beiden Löcher in den Gelenkarmen, die durch den Stift verbunden werden sollen, zur Überdeckung gebracht werden. Zudem ist schwer zu erkennen, welches der richtige Winkel ist, in dem das Gelenk arretiert werden soll. Daher kann es zu Arretierungen bei falschen Winkeln kommen.

Wird der Stift aus dem Gelenk gezogen, um von der Arretierstellung oder der Winkelbegrenzungsstellung in die Freigabestellung zu gelangen, geht die Information über den vorher eingestellten Winkel verloren. Dieser muss für den Fall, dass das Gelenk wieder in die Arretier- oder Winkelbegrenzungsstellung gebracht werden soll, mühsam durch Einstecken des Stiftes in das entsprechende Loch eingestellt werden.

Die DE 199 04 554 A1 offenbart ein Gelenk, bei dem der Winkelbereich, in dem der erste Gelenkarm gegen den zweiten Gelenkarm verschwenkbar ist, stufenlos eingestellt werden kann. Dazu sind zwei Scheiben vorgesehen, die drehfest mit einem Gelenkarm verbunden sind und die jeweils einen bogenförmigen Längsschlitz aufweisen. Am anderen Gelenkarm ist ein Querstift befestigt, der in die beiden Längsschlitze eingreift. Die beiden Scheiben können gegeneinander durch Drehung um die Schwenkachse des Gelenks verschoben werden. Dadurch wird der Bereich, in dem die beiden Schlitze zur Überdeckung gebracht sind und in dem sich der Querstift und damit der andere Gelenkarm bewegen kann, verändert.

Nachteilig ist, dass das in dieser Druckschrift beschriebene Gelenk nicht einfach in eine Arretierstellung oder eine Freigabestellung gebracht werden kann. Dazu müssen die Scheiben derart gegeneinander verschoben werden, dass der Bereich der Überdeckung der beiden Schlitze minimal bzw. maximal ist. Da die Scheiben über Drehung einer Schnecke verschoben werden, die in an der Scheibe vorgesehene Zähne eingreift, ist diese Umstellung mühsam und langwierig. Zudem ist auch in diesem Fall die Information über den vorher eingestellten Winkelbereich verloren.

Die DE 199 33 197 offenbart ein Gelenk, bei dem die beiden Gelenkarme über einen einstellbaren Winkelbereich gegeneinander verschwenkbar sind. Dafür sind in Abstände von jeweils 15° Löcher in Platten vorgesehen, in die Anschlagelemente eingesteckt werden können. Dies kann in beiden Schwenkrichtungen geschehen, so dass der mögliche Schwenkbereich durch die Anschlagelemente in beiden Richtungen eingeschränkt wird. Der Winkel zwischen den beiden Anschlagflächen, die an den jeweiligen Anschlagelementen anschlagen, können nur über einen Bereich von 15° stufenlos verstellt werden. Auf diese Weise ist es möglich, den Schwenkbereich, in dem die beiden Gelenkarme relativ zueinander verschwenkbar sind, stufenlos einzustellen.

Eine Arretierung der beiden Gelenkarm ist nur dadurch möglich, dass der Schwenkbereich so gewählt wird, dass die Anschlagsflächen auf beiden Seiten an die jeweiligen Anschlagsselemente anschlagen. Eine Arretierung der beiden Gelenkarme relativ zueinander ist folglich nur in Abständen von jeweils 15° möglich.

Der Erfindung liegt daher die Aufgabe zugrunde, ein Gelenk zur Verfügung zu stellen, dessen Arretierstellung einfach reproduzierbar einstellbar ist, und das über eine Einstellmöglichkeit verfügt, in der die Bewegung bis zu einem bestimmten Anschlagwinkel ermöglicht ist.

Die Erfindung löst das Problem durch ein gattungsgemäßes Gelenk, das eine Arretiereinrichtung besitzt, die durch Bewegen eines Riegels in eine Arretierstellung, in der der erste Gelenkarm und der zweite Gelenkarm relativ zueinander arretiert sind, eine Freigabestellung, in der der erste Gelenkarm und der zweite Gelenkarm frei relativ zueinander schwenkbar sind, und eine Winkelbegrenzungsstellung, in der der erste Gelenkarm und der zweite Gelenkarm bis zu einem voreingestellten Anschlag-Spreizwinkel gegeneinander schwenkbar sind und eine Verschwenkung zu größeren Spreizwinkein blockiert ist, bringbar ist.

Vorteilhaft ist, dass der Anschlag-Spreizwinkel einmal auf den gewünschten Wert eingestellt und danach nur noch die Stellung der Arretiereinrichtung verändert werden muss, um zwischen Arretierstellung, Freigabestellung und Winkelbegrenzungsstellung umzuschalten. Der voreingestellte Anschlag-Spreizwinkel bleibt dabei eingestellt, so dass auch ein Umschalten von der Freigabestellung in die Winkelbegrenzungsstellung oder Arretierstellung der Arretiereinrichtung einfach, schnell und reproduzierbar möglich ist.

Bevorzugt umfasst die Arretiervorrichtung eine Anschlag-Spreizwinkelverstelleinrichtung, die festlegbar drehfest am ersten Gelenkarm angeordnet ist und einen Anschlag, beispielsweise in Form einer Anschlagsnase, und eine Ausnehmung besitzt, und einen am zweiten Gelenkarm angeordneten Riegel umfasst und durch Einbringen des Riegels in die Ausnehmung in die Arretierstellung, durch Beabstanden des Riegels von der Ausnehmung in die Winkelbegrenzungsstellung und durch weiteres Beabstanden des Riegels von der Ausnehmung in die Freigabestellung bringbar ist.

Festlegbar drehfest meint insbesondere, dass die Anschlag-Spreizwinkelverstelleinrichtung drehfest am ersten Gelenkarm angeordnet ist, ihre Position relativ zum ersten Gelenkarm jedoch einstellbar ist.

Die Ausnehmung in der Anschlag-Spreizwinkelverstelleinrichtung kann dabei beispielsweise als Nut oder Bohrung ausgebildet sein. Die Bezeichnung "Riegel" meint hier keine bestimmte geometrische Form oder eine bestimmte Bewegungsrichtung, in die der Riegel von der Ausnehmung beabstandet wird. Durch die Bezeichnung "Riegel" wird lediglich das Funktionsprinzip des Bauteils benannt. Das Beabstanden des Riegels von der Ausnehmung kann in bezüglich der Schwenkachse (A) radialer oder axialer Richtung erfolgen. Zum Verändern der Position des Riegels ist ein Einstellelement am zweiten Gelenkarm vorgesehen, das beispielsweise ein Schieberegler ist, mit dem der Riegel in die verschiedenen Positionen gebracht werden kann.

In einer anderen Ausführungsform umfasst die Arretiereinrichtung eine Anschlag-Spreizwinkelverstelleinrichtung, wobei ein Riegel festlegbar drehfest am ersten Gelenkarm angeordnet ist, und einen am zweiten Gelenkarm angeordneten Anschlag und eine Ausnehmung. Durch Begrenzen des Riegels durch die Ausnehmung bzw. durch Ineingriffbringen des Riegels mit der Ausnehmung ist die Anschlag-Spreizwinkelverstelleinrichtung in die Arretierstellung, durch Beabstanden der Ausnehmung von dem Riegel in die Winkelbegrenzungsstellung und durch weiteres Beabstanden der Ausnehmung von dem Riegel in die Freigabestellung bringbar.

In anderen Worten ist in dieser Variante der Riegel drehfest an der Spreizwinkelverstelleinrichtung angeordnet. Zur Verstellung wird der Anschlag und/oder die Ausnehmung relativ zum Riegel verfahren.

Vorteilhafterweise ist die Anschlag-Spreizwinkelverstelleinrichtung als eine Zwischenscheibe mit einer im Wesentlichen kreisförmigen Oberseite, einer zur Oberseite im Wesentlichen parallelen im Wesentlichen kreisförmigen Unterseite und einer Umfangsfläche, an der der Anschlag und die Ausnehmung angeordnet ist, ausgebildet.

Vorteilhafterweise liegt dann, wenn die Arretiereinrichtung in der Winkelbegrenzungsstellung ist, und der erste Gelenkarm und der zweite Gelenkarm den Anschlag-Spreizwinkel einschließen, der Riegel an dem Anschlag an, so dass ein Schwenken des ersten Gelenkarmes relativ zum zweiten Gelenkarm über den Anschlag-Spreizwinkel hinaus blockiert ist. Ein Schwenken zu kleineren Winkeln ist möglich und wird durch die Position des Riegels relativ zum Anschlag nicht beeinflusst. Dies bedeutet, dass die Arretiereinrichtung in der Winkelbegrenzungsstellung ist, wenn der Riegel beim Schwenken des ersten Gelenkarmes relativ zum zweiten Gelenkarm an dem Anschlag anschlägt, wenn der Spreizwinkel über den Anschlag-Spreizwinkel hinaus vergrößert werden soll.

Vorteilhafterweise greift der Riegel im Wesentlichen spielfrei in die Ausnehmung ein, wenn die Arretiervorrichtung in der Arretierstellung ist. Im Wesentlichen spielfrei bedeutet, dass es nicht notwendig ist, dass der Riegel im mathematischen Sinne spielfrei ist. Es ist beispielsweise ausreichend, dass der mögliche Schwenkwinkel des ersten Gelenkarmes relativ zum zweiten Gelenkarm beispielsweise weniger als 1° beträgt, wenn die Arretiereinrichtung in der Arretierstellung ist.

In einer bevorzugten Ausführungsform weist die Zwischenscheibe zumindest über einen Teil ihres Umfanges Zähne auf und die Arretiervorrichtung umfasst eine Schnecke, die an dem ersten Gelenkarm angeordnet ist und mit den Zähnen der Zwischenscheibe kämmt. Besonders vorteilhafterweise kämmt die Schnecke mit den Zähnen der Zwischenscheibe selbsthemmend. Dadurch wird gewährleistet, dass die Zwischenscheibe einstellbar drehfest am ersten Gelenkarm gelagert ist. Über eine Drehung der Schnecke wird die Position der Zwischenscheibe relativ zum ersten Gelenkarm und damit auch die Position des Anschlags und der Ausnehmung der Zwischenscheibe und somit der Anschlag-Spreizwinkel stufenlos verändert. Durch die drehfeste Lagerung der Zwischenscheibe am ersten Gelenkarm ist sichergestellt, dass sich der so eingestellte Anschlag-Spreizwinkel nicht verändert, solange die Schnecke nicht betätigt wird. Um zu verhindern, dass die Schnecke unbeabsichtigt gedreht wird, ist vorteilhafterweise für die Drehung der Schnecke ein Werkzeug, beispielsweise ein Inbusschlüssel nötig. Dafür ist ein Ende einer Welle, auf der die Schnecke drehfest gelagert ist, mit einem Formschlusselement, beispielsweise einem Innensechskant, ausgestattet.

Vorteilhafterweise ist der Anschlag durch einen von der Schwenkachse (A) aus radial hervorstehenden Bereich der Zwischenscheibe gebildet. Dabei ist vorzugsweise die Seite des Anschlags, die für den Anschlag des Riegels vorgesehen ist, gerade ausgebildet. Dadurch entstehen beim Anschlagen des Riegels an dem Anschlag keine radialen Kräfte.

Die in Umfangsrichtung entgegengesetzte Seite des Anschlags ist rampenförmig ausgebildet. Dies bedeutet, die radiale Ausdehnung der Zwischenscheibe nimmt in diesem Bereich stetig zu. Dadurch ist gewährleistet, dass für den Fall, dass die Arretiereinrichtung von der Freigabestellung in die Winkelbegrenzungsstellung gebracht wird, während der Spreizwinkel zwischen dem ersten Gelenkarm und dem zweiten Gelenkarm größer ist als der eingestellte Anschlag-Spreizwinkel, ein Verschwenken des ersten Gelenkarmes relativ zum zweiten Gelenkarm über den Bereich des voreingestellten Anschlag-Spreizwinkel hinaus möglich ist, und der Riegel dabei leicht aus der Winkelbegrenzungsposition geschoben wird.

Vorzugsweise ist der Riegel mit einer Federkonstruktion ausgestattet, so dass er in diesem Fall nach dem Passieren des Anschlag-Spreizwinkels in die Winkelbegrenzungsposition zurückschnappt.

Vorteilhafterweise schließen sich die Ausnehmung und der Anschlag entlang des Umfangs der Zwischenscheibe direkt aneinander an. Dies gewährleistet auf besonders einfache Weise, dass der Anschlag-Spreizwinkel auch derjenige Spreizwinkel ist, in dem das Gelenk arretierbar ist.

In einer bevorzugten Ausgestaltung umfasst das Gelenk eine zweite Arretiereinrichtung, die in eine Winkelbegrenzungsstellung bringbar ist, in der der erste Gelenkarm und der zweite Gelenkarm bis zu einem voreingestellten zweiten Anschlag-Spreizwinkel gegeneinander schwenkbar sind und eine Verschwenkung zu kleineren Spreizwinkeln blockiert ist. Dabei ist vorzugsweise vorgesehen, dass auch die zweite Arretiereinrichtung eine Zwischenscheibe aufweist, die mit einem dafür vorgesehenen zweiten Riegel in eine Winkelbegrenzungsstellung bringbar ist. Dieser zweite Riegel ist vorzugsweise wie der erste Riegel am zweiten Gelenkarm vorgesehen. Auch die zweite Zwischenscheibe ist über eine dafür vorgesehene zweite Schnecke stufenlos relativ zum ersten Gelenkarm verstellbar. Da die zweite Arretiereinrichtung in der Winkelbegrenzungsstellung die Verschwenkung des ersten Gelenkarms relativ zum zweiten Gelenkarm zu kleineren Winkeln als dem voreingestellten zweiten Anschlag-Spreizwinkel verhindert, kann so der Bereich, in dem der erste Gelenkarm relativ zum zweiten Gelenkarm schwenkbar ist, auf beiden Seiten stufenlos begrenzt werden. Natürlich können auch die beiden Riegel und die beiden Schnecken an jeweils unterschiedlichen Gelenkarmen vorgesehen sein, solange Riegel und Schnecke, die zu einer Arretiereinrichtung gehören, an verschiedenen Gelenkarmen angeordnet sind.

Vorteilhafterweise weist eine Orthese eine erste Aufnahmeeinrichtung für ein erstes Körperglied und eine zweite Aufnahmeeinrichtung für ein zweites Körperglied auf, die durch mindestens ein oben beschriebenes Gelenk gegeneinander schwenkbar verbunden sind und einen Orthesenwinkel einschließen. Eine derartige Orthese hat den Vorteil, dass zum Anlegen der Orthese das Gelenk in die Freigabestellung bringbar ist, so dass die Orthese bequem und einfach anlegbar ist und die Orthese dann schnell, einfach und reproduzierbar entweder in einem bestimmten Orthesenwinkel arretierbar ist oder ein Schwenken der ersten Aufnahmeeinrichtung relativ zur zweiten Aufnahmeeinrichtung bis zum voreingestellten Anschlag-Spreizwinkel erlaubt. Das Umstellen erfolgt dabei einfach dadurch, dass der Riegel der Arretiereinrichtung in die jeweils gewünschte Position gebracht wird. Dabei kann der Anschlag-Spreizwinkel voreingestellt werden, bevor die Orthese angelegt wird.

Vorzugsweise sind die erste Aufnahmeeinrichtung und die zweite Aufnahmeeinrichtung mit mindestens einem zweiten oben beschriebenen Gelenk verbunden und das erste Gelenk begrenzt in der Winkelbegrenzungsstellung das Schwenken der ersten Aufnahmeeinrichtung relativ zur zweiten Aufnahmeeinrichtung in einer Richtung und das zweite Gelenk begrenzt in der Winkelbegrenzungsstellung das Schwenken der ersten Aufnahmeeinrichtung relativ zur zweiten Aufnahmeeinrichtung in einer anderen Richtung. Wenn sich beide Gelenke in der jeweiligen Winkelbegrenzungsstellung befinden, ist daher ein Schwenken der ersten Aufnahmeeinrichtung relativ zur zweiten Aufnahmeeinrichtung nur zwischen den beiden voreingestellten Anschlag-Spreizwinkeln der beiden Gelenke möglich.

Besonders vorteilhafterweise handelt es sich bei der Orthese um eine Knie- oder Ellenbogenorthese.

Mit Hilfe einer Zeichnung wird nun ein bevorzugtes Ausführungsbeispiel der Erfindung beschrieben. Es zeigt:
- Figur 1: die Seitenansicht eines erfindungsgemäßen Gelenkes,
- Figur 2: eine Draufsicht auf das Gelenk nach Figur 1,
- Figur 3a: eine Draufsicht auf die Arretiereinrichtung des Gelenks nach Figur 1 und 2 in der Arretierstellung,
- Figur 3b: eine Draufsicht auf die Arretiereinrichtung nach Figur 3a in der Winkelbegrenzungsstellung,
- Figur 3c: eine Draufsicht auf die Arretiereinrichtung nach Figur 3a und 3b in der Freigabestellung und
- Figur 4: eine Draufsicht auf eine Arretiereinrichtung nach Figur 3b, bei der der Spreizwinkel nicht der Anschlag-Spreizwinkel ist.
- Figur 5: zeigt eine schematische Draufsicht auf eine Arretiereinrichtung nach einer anderen Ausführungsform der Erfindung

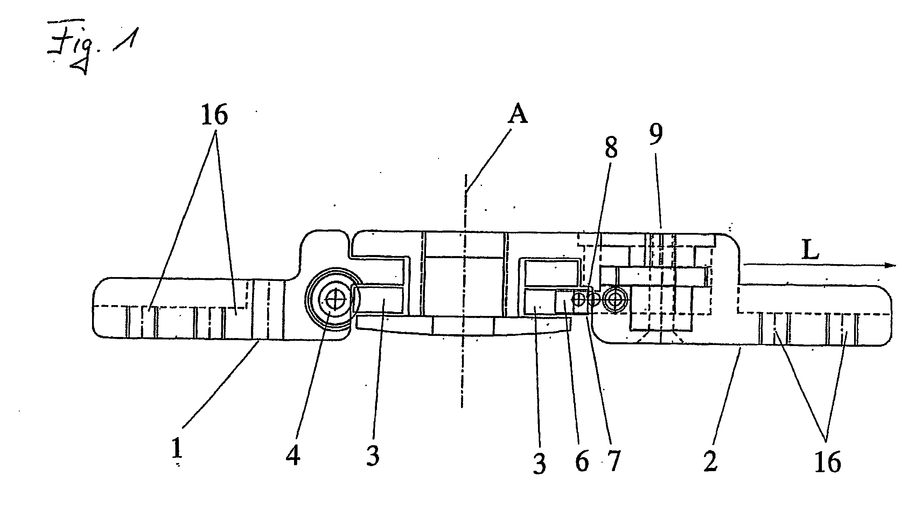

Figur 1 zeigt eine Seitenansicht eines Gelenkes einer bevorzugten Ausführungsform der Erfindung. Ein erster Gelenkarm 1 und ein zweiter Gelenkarm 2 sind um eine Schwenkachse A schwenkbar aneinander gelagert. Die beiden Gelenkarme weisen Verbindungsvorrichtungen 16 auf. Darüber sind sie beispielsweise mit Manschetten verbindbar, die an verschiedene Körperteile anlegbar sind. Die Manschetten können dabei beispielsweise über Klettverschlüsse oder andere reversibel verschließbare Befestigungsmittel an dem jeweiligen Körperteil befestigbar sein. Dabei handelt es sich beispielsweise um einen Ober- oder einen Unterarm. Die Manschetten weisen Verbindungsmittel auf, die sie mit den Verbindungsmitteln 16 an den Gelenkarmen 1, 2 verbindbar machen.

Am ersten Gelenkarm 1 ist eine Zwischenscheibe 3 einstellbar drehfest gelagert. Die Zwischenscheibe 3 hat einen im Wesentlichen scheibenförmigen Grundkörper mit einer Oberseite, einer im Wesentlichen zu der Oberseite parallelen Unterseite und einer zwischen der Oberseite und der Unterseite angeordneten Umfangsfläche. Die Oberseite und die Unterseite der Zwischenscheibe 3 sind im Wesentlichen kreisförmig ausgebildet. Die Zwischenscheibe 3 weist eine zentrale Bohrung auf, durch deren Mittelpunkt die Schwenkachse A verläuft, wenn die Zwischenscheibe mit dem ersten Gelenkarm verbunden ist. Die Zwischenscheibe ist mit dem ersten Gelenkarm um die Schwenkachse A schwenkbar.

Die Zwischenscheibe 3 weist über zumindest einen Teil ihres Umfangs radial nach außen angeordnete Zähne 5 sowie einen Anschlag 7 in Form einer Anschlagsnase und eine Ausnehmung 6 auf.

In dem ersten Gelenkarm 1 ist eine Schnecke 4 angeordnet. Diese Schnecke ist drehfest auf eine Welle 11 gelagert, deren eines Ende beispielsweise mit einem Formschlusselement, wie einem Innensechskant, versehen ist. Die Schnecke 4 kämmt mit den Zähnen 5 der Zwischenscheibe 3. Durch Drehung der Welle 11 und damit der Schnecke 4 wird die Position der Zwischenscheibe 3 relativ zur Schnecke 4 verändert. Die Zwischenscheibe 3 wird dadurch um die Schwenkachse A gedreht. Dadurch wird auch die Position der Ausnehmung 6 und der Anschlagsnase 7 relativ zum ersten Gelenkarm 1 verändert. Diese Änderung erfolgt stufenlos, da auch die Schnecke 4 stufenlos drehbar ist.

Im zweiten Gelenkarm 2 ist ein Riegel 8 verschieblich angeordnet. Dieser Riegel ist über eine Einstelleinrichtung 9 verschiebbar, die ebenfalls am zweiten Gelenkarm 2 angeordnet ist. Die Verschiebung erfolgt dabei parallel zu der Längsrichtung L des zweiten Gelenkarmes 2.

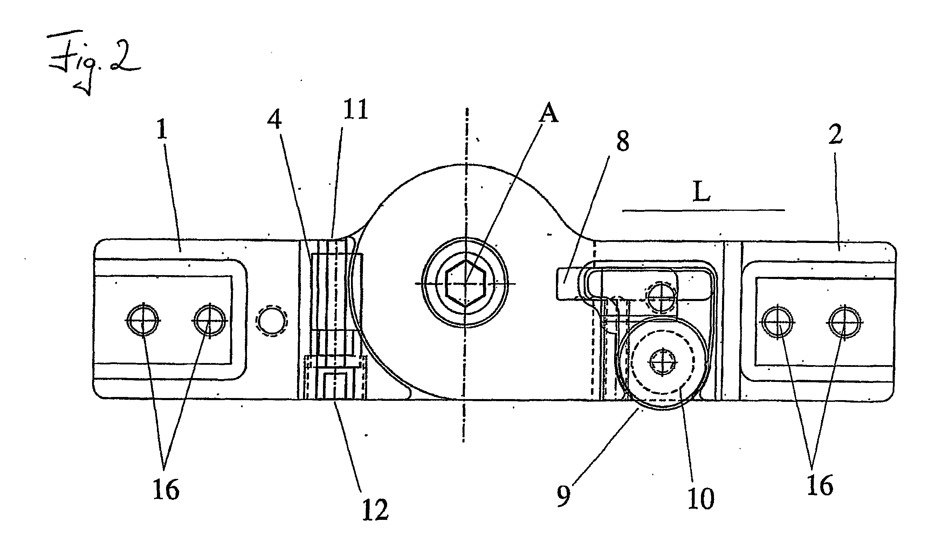

Figur 2 zeigt eine Draufsicht auf das in Figur 1 in Seitenansicht gezeigte Gelenk. Der erste Gelenkarm 1 und der zweite Gelenkarm 2 sind um die Schwenkachse A, die in Figur 2 aus der Bildebene herausragt, schwenkbar gelagert. Im zweiten Gelenkarm 2 ist der Riegel 8 durch die Einstelleinrichtung 9 in verschiedene Positionen bringbar. Die Einstelleinrichtung 9 umfasst ein Stellrad 10, dessen Rotationsbewegung in eine Bewegung des Riegels 8 radial zur Schwenkachse A umgesetzt wird.

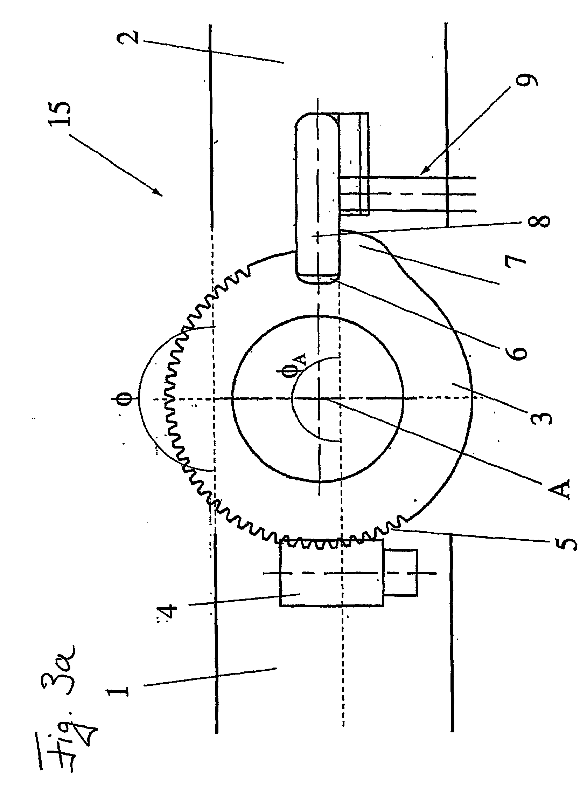

In den Figuren 3a bis 3c ist jeweils eine Arretiereinrichtung 15 in den verschiedenen Stellungen gezeigt. Es ist jeweils die Zwischenscheibe 3 abgebildet, die zumindest entlang eines Teils ihres Umfanges die Zähne 5 aufweist, in die die Schnecke 4 eingreift.

In Figur 3a sind der erste und der zweite Gelenkarm 1, 2 durch dicke durchgezogene Linien angedeutet. Zwischen den beiden Gelenkarmen 1, 2 ist der Spreizwinkel ϕ eingeschlossen. Zwischen der Anschlagsnase 7 und dem ersten Gelenkarm 1 ist der Anschlag-Spreizwinkel Φ_{A} eingeschlossen. In Figur 3a sind sowohl die beiden Gelenkarme 1,2 als auch die Kante der Anschlagsnase 7, an der der Riegel 8 anschlägt, parallel. Daher sind die Winkel Φ, Φ_{A} für eine bessere Übersichtlichkeit gemeinsam eingezeichnet. Im gezeigten Beispiel betragen beide Winkel 180°.

In Figur 3a befindet sich die Arretiereinrichtung in der Arretierstellung. Der Riegel 8 greift in der Ausnehmung 6 der Zwischenscheibe 3 ein. Ein Schwenken der Zwischenscheibe 3 relativ zum Riegel 8 ist nicht möglich. Da die Zwischenscheibe drehfest am ersten Gelenkarm 1 und der Riegel 8 drehfest am zweiten Gelenkarm 2 angeordnet sind, ist auch ein Verschwenken der beiden Gelenkarme 1, 2 relativ zueinander nicht möglich.

Das Drehen an der Welle 11, an der die Schnecke 4 drehfest gelagert ist, hat eine Drehung der Zwischenscheibe 3 relativ zur Schnecke 4 zur Folge. Damit ändert sich auch die Position der Ausnehmung 6 und der Anschlagsnase 7 relativ zur Schnecke und damit zum ersten Gelenkarm 1, an dem die Schnecke 4 befestigt ist. In der Arretierstellung der Arretiereinrichtung 15 greift der Riegel 8 in die Ausnehmung 6 ein. Daher ändert sich auch die Position des zweiten Gelenkarmes 2 relativ zum ersten Gelenkarm 1, wenn die Schnecke gedreht wird. Auf diese Weise wird der Winkel geändert, in dem das Gelenk arretiert ist.

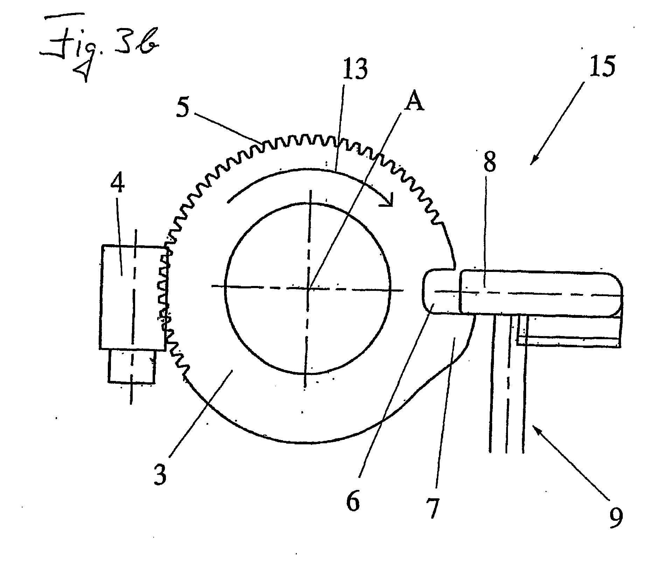

In Figur 3b befindet sich die Arretiereinrichtung 15 in der Winkelbegrenzungsstellung. Zwischen dem ersten Gelenkarm 1 und dem zweiten Gelenkarm 2 (beide nicht gezeigt) ist der Anschlag-Spreizwinkel Φ_{A} eingeschlossen. Daher liegt der Riegel 8 an der Anschlagsnase 7 an. Ein Schwenken der Zwischenscheibe relativ zum Riegel 8 ist in Figur 3b nur in eine Richtung, nämlich zu kleineren Spreizwinketn Φ möglich. Dies ist in Figur 3b durch den Pfeil 13 angedeutet. In die entgegengesetzte Richtung wird das Schwenken durch den Anschlag des Riegels 8 an der Anschlagsnase 7 verhindert. In der gezeigten Stellung betragen sowohl der Spreizwinkel Φ, als auch der Anschlag-Spreizwinkel Φ_{A} 180°. Der Übersichtlichkeit wegen sind beide nicht eingezeichnet.

Durch Drehen der Schnecke 4 wird wie in der in Figur 3a gezeigten Arretierstellung die Position der Zwischenscheibe 3 relativ zum ersten Gelenkarm 1 verändert. Durch die damit verbundene Rotation der Zwischenscheibe 3 um die Schwenkachse A ändert sich auch der zwischen der Anschlagsnase 7 und dem ersten Gelenkarm eingeschlossene Anschlag-Spreizwinkel Φ_{A}.

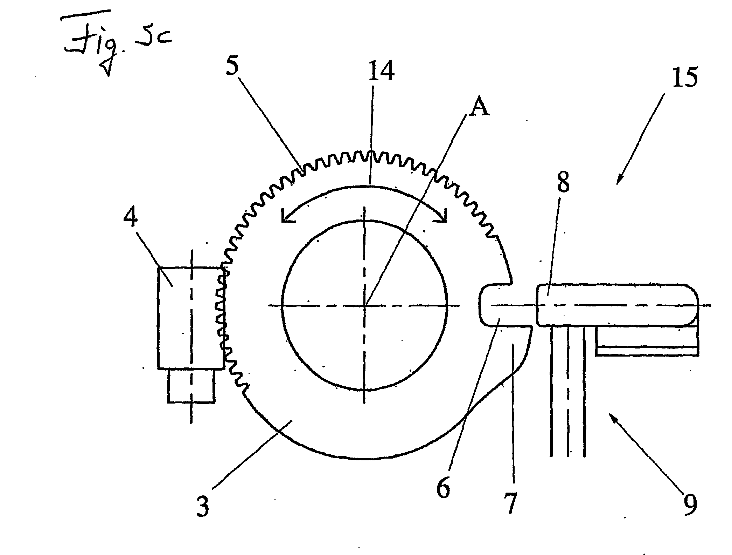

Figur 3c zeigt eine Arretiereinrichtung 15 in der Freigabestellung. Ein Verschwenken des ersten Gelenkarmes 1 relativ zum zweiten Gelenkarm 2 ist in beide Richtungen frei möglich. Dies ist in Figur 3c durch den Doppelpfeil 14 angedeutet. Eine Drehung der Schnecke 4 ändert zwar die Position der Anschlagsnase 7 relativ zum ersten Gelenkarm 1, hat jedoch auf die Schwenkbarkeit des ersten Gelenkarmes 1 relativ zum zweiten Gelenkarm 2 keinen Einfluss. Mögliche Spreizwinkel Φ sind sowohl Winkel größer als auch kleiner als der voreingestellte Anschlag-Spreizwinkel Φ_{A}. Wird jedoch die Arretiereinrichtung 15 aus der Freigabestellung wieder in die Winkelbegrenzungsstellung oder die Arretierstellung gebracht, ist die Begrenzung der möglichen Schwenkbewegung durch die Anschlagsnase 7 bzw. die Ausnehmung 6 wieder gegeben, ohne dass der Anschlag-Spreizwinkel Φ_{A} neu eingestellt werden müsste.

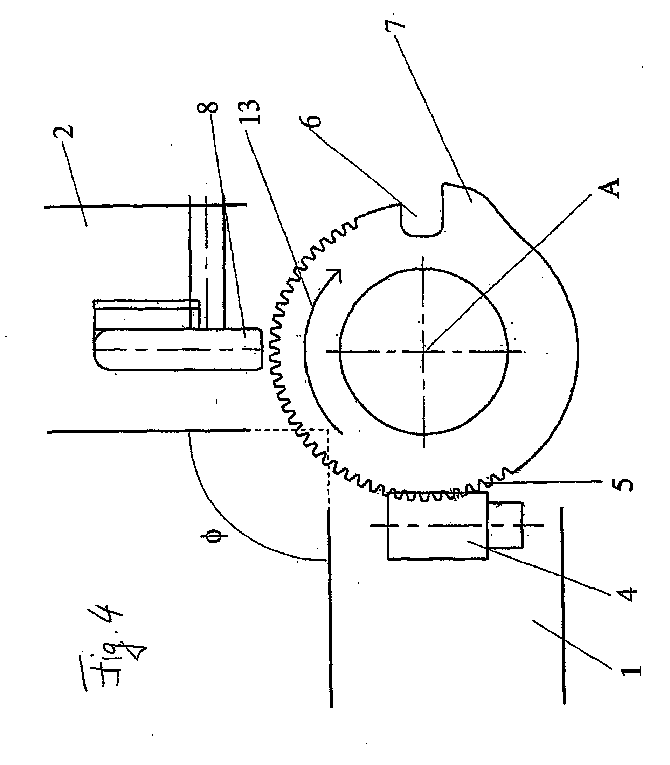

Figur 4 zeigt eine Arretiereinrichtung 15 in der Winkelbegrenzungsstellung. Wie in Figur 3b beträgt der Anschlag-Spreizwinkel Φ_{A} 180°, da die Position der Zwischenscheibe 3 relativ zur Schnecke 4 und damit zum ersten Gelenkarm 1 unverändert ist. Der erste Gelenkarm 1 und der zweite Gelenkarm 2 sind in Figur 4 durch dicke durchgezogene Linien angedeutet. In Figur 4 ist der zweite Gelenkarm 2 gegen den ersten Gelenkarm 1 verschwenkt. Dies ist nur zu Spreizwinkeln Φ möglich, die kleiner sind als der voreingestellte Anschlag-Spreizwinkel Φ_{A} Der zwischen dem ersten Gelenkarm 1 und dem zweiten Gelenkarm 2 eingeschlossene Spreizwinkel Φ beträgt in Figur 4 90°.

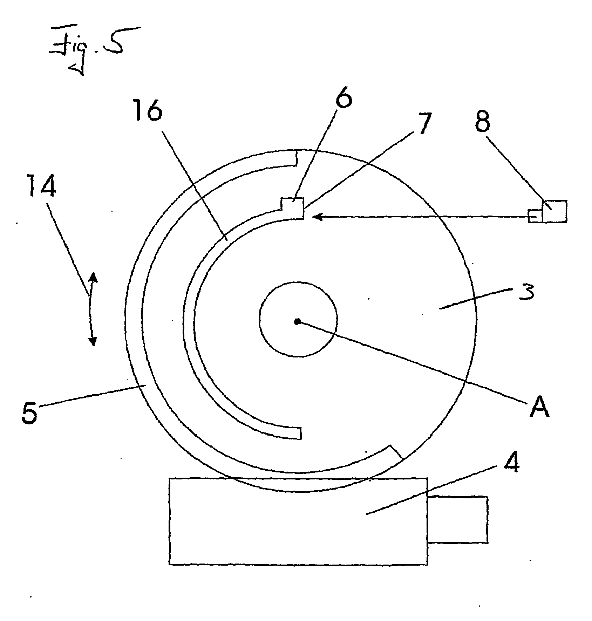

Figur 5 zeigt eine Draufsicht auf eine Arretiereinrichtung 15. Es ist eine Zwischenscheibe 3 gezeigt, an deren Rand sich die Zähne 5 befinden, die in Figur 5 durch einen Streifen angedeutet ist. Die Zähne 5 greifen in eine Schnecke 4 ein, so dass die Position der Zwischenscheibe 3 relativ zu dem Gelenkarm, an dem sie angeordnet ist, verstellt werden kann. Der Gelenkarm ist in Figur 5 nicht gezeigt.

Bei der in Figur 5 gezeigten Ausführungsform weist die Zwischenscheibe 3 einen zur Schwenkachse konzentrisch verlaufenden Schlitz 16 auf. Der Schlitz 16 besitzt an seinem einen Ende die Ausnehmung 6. Eine in Drehrichtung randständige Begrenzung von Schlitz 16 bildet den Anschlag 7. Der Riegel 8 ist in Richtung parallel zur Schwenkachse A beweglich gelagert. In Figur 5 erfolgt die Bewegung des Riegels 8 also aus der Zeichenebene hinaus, bzw. in sie hinein.

Ist der Riegel 8 in seiner abgesenkten Position, greift er mit dem in Figur 5 rechten Anteil in die Ausnehmung 6 ein. Dieser Anteil des Riegels 8 ist so ausgebildet, dass er für einen Eingriff in den Schlitz 16 jenseits der Ausnehmung 6 zu groß ist. Eine Bewegung der Zwischenscheibe 3 relativ zum Riegel 8 ist in dieser Position des Riegels 8 nicht möglich. Die Arretiereinrichtung 15 befindet daher sich in der Arretierstellung.

Durch Beabstanden des Riegels 8 von der Ausnehmung 6, im gezeigten Beispiel also durch Bewegung des Riegels 8 nach oben, greift der Riegel 8 nur noch mit seinem in Figur 5 linken Anteil in den Schlitz 16 ein. Die Zwischenscheibe 3 kann nur relativ zum Riegel 8 geschwenkt werden. Erst wenn der vorher eingestellte, nicht gezeigte, Anschlag-Spreizwinkel erreicht ist, schlägt der Riegel 8 an dem Anschlag 7 an und eine Bewegung über diesen Spreizwinkel hinaus ist nicht möglich. Die Arretiereinrichtung 15 befindet sich in der Winkelbegrenzungsstellung.

Weiteres Beabstanden des Riegels 8 von der Ausnehmung 6, also in Figur 5 eine weitere Bewegung des Riegels 8 nach oben, hat zur Folge, dass Riegel 8 und Zwischenscheibe 3 in keiner Wirkverbindung mehr stehen. Ein Schwenken der Zwischenscheibe 3 relativ zum Riegel 8 und damit auch der beiden mit diesen Teilen drehfest verbundenen Gelenkarmen ist uneingeschränkt möglich. Die Arretiereinrichtung 15 befindet sich in der Freigabestellung.

### Bezugszeichenliste

- A: Schwenkachse
- Φ: Spreizwinkel
- Φ_{A}: Anschlag-Spreizwinkel

- 1: erster Gelenkarm
- 2: zweiter Gelenkarm
- 3: Zwischenscheibe
- 4: Schnecke
- 5: Zähne
- 6: Ausnehmung
- 7: Anschlag
- 8: Riegel
- 9: Einstelleinrichtung
- 10: Stellrad
- 11: Welle
- 12: Formschlusselement
- 13: Pfeil
- 14: Doppelpfeil
- 15: Arretiereinrichtung
- 16: Schlitz

## Patentansprüche

1. Gelenk mit
(a) einem ersten Gelenkarm (1),
(b) einem zweiten Gelenkarm (2),
- der um eine Schwenkachse (A) schwenkbar an dem ersten Gelenkarm (1) gelagert ist und
- mit dem ersten Gelenkarm (1) einen Spreizwinkel (Φ) einschließt, der zwischen einem maximalen Spreizwinkel (Φₘₐₓ) und einem minimalen Spreizwinkel (Φₘᵢₙ) liegt, die durch die Bauart des Gelenks bestimmt sind, und
(c) einer Arretiereinrichtung (15), die durch Bewegen eines Riegels (8) wahlweise in
- eine Arretierstellung, in der der erste Gelenkarm (1) und der zweite Gelenkarm (2) relativ zueinander arretiert sind, und
- eine Winkelbegrenzungsstellung, in der
(i) der erste Gelenkarm (1) und der zweite Gelenkarm (2) bis zu einem voreingestellten Anschlag-Spreizwinkel (Φ_{A}) gegeneinander schwenkbar sind und
(ii) eine Verschwenkung zu größeren Spreizwinkeln (Φ) blockiert ist,
bringbar ist,
**dadurch gekennzeichnet, dass**
(d) die Arretiereinrichtung (15) durch Bewegen des Riegels (8) in eine Freigabestellung bringbar ist, in der der erste Gelenkarm (1) und der zweite Gelenkarm (2) frei relativ zueinander schwenkbar sind.

2. Gelenk nach Anspruch 1, **dadurch gekennzeichnet, dass** die Arretiereinrichtung (15)
(i) eine Anschlag-Spreizwinkelverstelleinrichtung (3),
- die festlegbar drehfest am ersten Gelenkarm (1) angeordnet ist und
- einen Anschlag (7) und
- eine Ausnehmung (6) besitzt,
und
(ii) den am zweiten Gelenkarm (2) angeordneten Riegel (8) umfasst und
(iii) durch Einbringen des Riegels (8) in die Ausnehmung (6) in die Arretierstellung,
durch Beabstanden des Riegels (8) von der Ausnehmung (6) in die Winkelbegrenzungsstellung und
durch weiteres Beabstanden des Riegels (8) von der Ausnehmung (6) in die Freigabestellung bringbar ist.

3. Gelenk nach Anspruch 1, **dadurch gekennzeichnet, dass** die Arretiereinrichtung (15)
(i) eine Anschlag-Spreizwinkelverstelleinrichtung (3),
- wobei der Riegel (8) festlegbar drehfest am ersten Gelenkarm (1) angeordnet ist,
(ii) und einen am zweiten Gelenkarm (2) angeordneten
- Anschlag (7) und
- eine Ausnehmung (6) besitzt,
(iii) und die durch Ineingriffbringen des Riegels (8) mit der Ausnehmung (6) in die Arretierstellung,
durch Beabstanden der Ausnehmung (6) von dem Riegel (8) in die Winkelbegrenzungsstellung und
durch weiteres Beabstanden der Ausnehmung (6) von dem Riegel (8) in die Freigabestellung bringbar ist.

4. Gelenk nach Anspruch 2 oder 3, **dadurch gekennzeichnet, dass** die Anschlag-Spreizwinkelverstelleinrichtung (3) als Zwischenscheibe (3) mit
- einer im Wesentlichen kreisförmigen Oberseite
- einer zur Oberseite im Wesentlichen parallelen im Wesentlichen kreisförmigen Unterseite und
- einer Umfangsfläche, an der
der Anschlag (7) und die Ausnehmung (6)
oder der Riegel (8) angeordnet ist,
ausgebildet ist.

5. Gelenk nach einem der Ansprüche 2, 3 oder 4, **dadurch gekennzeichnet, dass** dann, wenn
- die Arretiereinrichtung (15) in der Winkelbegrenzungsstellung ist und
- der erste Gelenkarm (1) und der zweiten Gelenkarm (2) den Anschlags-Spreizwinkel ϕ_{A} einschließen,
der Riegel (8) an dem Anschlag (7) anliegt, so dass ein Schwenken des ersten Gelenkarmes (1) relativ zum zweiten Gelenkarm (2) über den Anschlags-Spreizwinkel (Φ_{A}) hinaus blockiert ist.

6. Gelenk nach einem der Ansprüche 2 bis 5, **dadurch gekennzeichnet, dass** der Riegel (8) im Wesentlichen spielfrei in die Ausnehmung (6) eingreift, wenn die Arretiervorrichtung in der Arretierstellung ist.

7. Gelenk nach einem Ansprüche 2 bis 6, **dadurch gekennzeichnet, dass**
- die Zwischenscheibe (3) zumindest über einen Teil ihres Umfanges Zähne (5) aufweist, und
- die Arretiervorrichtung eine Schnecke (4) umfasst, die an dem ersten Gelenkarm angeordnet ist und mit den Zähnen (5) der Zwischenscheibe (3) kämmt.

8. Gelenk nach Anspruch 7, **dadurch gekennzeichnet, dass** die Schnecke (4) mit den Zähnen (5) der Zwischenscheibe (3) selbsthemmend kämmt.

9. Gelenk nach einem der Ansprüche 2 bis 8, **dadurch gekennzeichnet, dass** der Anschlag (7) durch einen von der Schwenkachse (A) aus radial hervorstehenden Bereich der Zwischenscheibe (3) gebildet ist.

10. Gelenk nach einem der Ansprüche 2 bis 9, **dadurch gekennzeichnet, dass** sich entlang des Umfangs der Zwischenscheibe (3)
- die Ausnehmung (6) und
- der Anschlag (7) direkt aneinander anschließen.

11. Gelenk nach einem der vorstehenden Ansprüche, **dadurch gekennzeichnet, dass** das Gelenk eine zweite Arretiereinrichtung (15) umfasst, die in eine Winkelbegrenzungsstellung bringbar ist, in der
- der erste Gelenkarm (1) und der zweite Gelenkarm (2) bis zu einem voreingestellten zweiten Anschlags-Spreizwinkel (γ_{A}) gegeneinander schwenkbar sind und
- eine Verschwenkung zu kleineren Spreizwinkeln (Φ) blockiert ist.

12. Orthese mit
(a) einer ersten Aufnahmeeinrichtung für ein erstes Körperglied und
(b) einer zweiten Aufnahmeeinrichtung für ein zweites Körperglied, die
- durch mindestens ein Gelenk nach einem der vorstehenden Ansprüche gegeneinander schwenkbar verbunden sind und
- einen Orthesenwinkel (θ) einschließen.

13. Orthese nach Anspruch 12, **dadurch gekennzeichnet, dass** die erste Aufnahmeeinrichtung und die zweite Aufnahmeeinrichtung mit mindestens einem zweiten Gelenk nach einem der Ansprüche 1 bis 11 verbunden sind und dass
- das erste Gelenk in der Winkelbegrenzungsstellung das Schwenken der ersten Aufnahmeeinrichtung relativ zur zweiten Aufnahmeeinrichtung in einer Richtung begrenzt und
- das zweite Gelenk in der Winkelbegrenzungsstellung das Schwenken der ersten Aufnahmeeinrichtung relativ zur zweiten Aufnahmeeinrichtung in einer anderen Richtung begrenzt.

14. Orthese nach Anspruch 12 oder 13, **dadurch gekennzeichnet, dass** sie eine Knie- oder Ellenbogenorthese ist.

## Claims

1. A joint with
(a) a first joint arm (1) and
(b) a second joint arm (2),
- which is mounted on the first joint arm (1) such that it can pivot about a pivot axis (A) and
- which includes a spread angle (Φ) with the first joint arm (1), which spread angle (Φ) lies between a maximum spread angle (Φₘₐₓ) and a minimum spread angle (Φₘᵢₙ), determined by the design of the joint, and
(c) a locking device (15) which, by movement of a bar (8), can selectively be brought into
- a locking position, in which the first joint arm (1) and the second joint arm (2) are locked relative to one another,
- an angle-limiting position, in which
(i) the first joint arm (1) and the second joint arm (2) can be pivoted with respect to one another up to a predetermined stop spread angle (Φ_{A}) and
(ii) pivoting to larger angles of spread (Φ) is blocked,
**characterized in that**
(d) the locking device (15) can be brought into a release position, in which the first joint arm (1) and the second joint arm (2) can be pivoted with respect to one another, by movement of the bar (8).

2. The joint as claimed in claim 1, **characterized in that** the locking device (15)
(i) comprises a stop spread angle adjustment device (3),
- which is arranged on the first joint arm (1) in a definably rotationally securable fashion and
- has a stop (7) and
- a recess (6),
and
(ii) comprises the bar (8) arranged on the second joint arm (2) and
(iii) can be brought into the locking position by inserting the bar (8) into the recess (6), can be brought into the angle-limiting position by spacing the bar (8) from the recess (6), and
can be brought into the release position by spacing the bar (8) further from the recess (6).

3. The joint as claimed in claim 1, **characterized in that** the locking device (15)
(i) comprises a stop spread angle adjustment device (3),
- wherein the bar (8) is arranged on the first joint arm (1) in a definably rotationally secured fashion,
(ii) and has, arranged on the second joint arm (2),
- a stop (7) and
- a recess (6),
(iii) and can be brought into the locking position by making the bar (8) and the recess (6) engage,
can be brought into the angle-limiting position by spacing the recess (6) from the bar (8), and can be brought into the release position by spacing the recess (6) further from the bar (8).

4. The joint as claimed in claim 2 or 3, **characterized in that** the stop spread angle adjustment device (3) is designed as intermediate disk (3) with
- a substantially circular upper side,
- a substantially circular lower side substantially parallel to the upper side, and
- a circumferential surface on which
- the stop (7) and the recess (6) or the bar (8) is arranged.

5. The joint as claimed in one of claims 2, 3 or 4, **characterized in that**, if
- the locking device (15) is in the angle-limiting position and
- the first joint arm (1) and the second joint arm (2) subtend the stop spread angle ϕ_{A},
the bar (8) butts against the stop (7) and so pivoting of the first joint arm (1) relative to the second joint arm (2) is blocked beyond the stop spread angle (Φ_{A}).

6. The joint as claimed in one of claims 2 to 5, **characterized in that** the bar (8) engages into the recess (6) substantially without play if the locking apparatus is in the locking position.

7. The joint as claimed in one of claims 2 to 6, **characterized in that**
- the intermediate disk (3) has teeth (5) over at least a part of its circumference and
- the locking apparatus comprises a worm (4), which is arranged on the first joint arm and meshes with the teeth (5) of the intermediate disk (3).

8. The joint as claimed in claim 7, **characterized in that** the worm (4) meshes with the teeth (5) of the intermediate disk (3) in a self-locking fashion.

9. The joint as claimed in one of claims 2 to 8, **characterized in that** the stop (7) is formed by a region of the intermediate disk (3) protruding radially from the pivot axis (A).

10. The joint as claimed in one of claims 2 to 9, **characterized in that**, along the circumference of the intermediate disk (3),
- the recess (6) and
- the stop (7) directly adjoin one another.

11. The joint as claimed in one of the preceding claims, **characterized in that** the joint comprises a second locking device (15), which can be brought into an angle-limiting position, in which
- the first joint arm (1) and the second joint arm (2) can be pivoted with respect to one another up to a predetermined second stop spread angle (γ_{A}) and
- pivoting to smaller angles of spread (Φ) is blocked.

12. An orthesis with
(a) a first receptacle device for a first body limb and
(b) a second receptacle device for a second body limb, which
- are connected to one another such that they can pivot by means of at least one joint as claimed in one of the preceding claims, and
- subtend an orthesis angle (θ).

13. The orthesis as claimed in claim 12, **characterized in that** the first receptacle device and the second receptacle device are connected by at least one second joint as claimed in one of claims 1 to 11 and **in that**
- the first joint limits the pivoting of the first receptacle device relative to the second receptacle device in one direction in the angle-limiting position, and
- the second joint limits the pivoting of the first receptacle device relative to the second receptacle device in another direction in the angle-limiting position.

14. The orthesis as claimed in claim 12 or 13, **characterized in that** it is a knee or elbow orthesis.

## Revendications

1. Articulation, comprenant
(a) un premier bras d'articulation (1),
(b) un second bras d'articulation (2),
- qui est monté sur le premier bras l'articulation (1) en pivotement autour d'un axe de pivotement (A), et
- qui définit avec le premier bras d'articulation (1) un angle d'écartement (Φ), qui tombe entre un angle d'écartement maximum (Φmax) et un angle d'écartement minimum (Φmin), lesquels sont déterminés par le mode de construction de l'articulation, et
(c) un moyen d'arrêt (15) qui, par déplacement d'un verrou (8), peut être amené sélectivement
- dans une position d'arrêt, dans laquelle le premier bras d'articulation (1) et le second bras d'articulation (2) sont arrêtés l'un par rapport à l'autre, et
- une position de limitation angulaire dans laquelle
(i) le premier bras d'articulation (1) et le second bras d'articulation (2) sont capables de pivoter l'un par rapport à l'autre jusqu'à un angle d'écartement de butée (ΦA) préétabli, et
(ii) un pivotement vers des angles d'écartement (F) plus élevés est bloqué,
**caractérisée en ce que**
(d) le moyen d'arrêt (15) est susceptible d'être amené, par déplacement du verrou (8), jusque dans une position de libération dans laquelle le premier bras articulation (1) et le second bras d'articulation (2) sont capables de pivoter librement l'un par rapport à l'autre.

2. Articulation selon la revendication 1, **caractérisée en ce que** le moyen d'arrêt (15) comprend
(i) un système de réglage d'angle d'écartement (3) à butée, lequel est agencé solidairement en rotation sur le premier bras d'articulation (1) de façon à pouvoir être immobilisé, et possède
- une butée (7) et
- un évidement (6),
et
(ii) le verrou (8) agencé sur le second bras d'articulation (2), et
(iii) peut être amené
- dans la position d'arrêt par introduction du verrou (8) dans l'évidement (6),
- dans la position de limitation angulaire en écartant le verrou (8) vis-à-vis de l'évidement (6), et
- dans la position de libération en poursuivant l'écartement du verrou (8) vis-à-vis de l'évidement (6).

3. Articulation selon la revendication 1, **caractérisée en ce que** le moyen d'arrêt (15) possède un moyen de réglage (3) de l'angle d'écartement de butée
- tel que le verrou (8) est agencé solidairement en rotation sur le premier bras d'articulation (1) de manière à pouvoir être immobilisé, (ii) et possède
une butée (7) et
- un évidement (6), agencé sur le second bras d'articulation (2),
(iii) et qui peut être amené
- dans la position d'arrêt par mise en engagement du verrou (8) avec l'évidement (6),
- dans la position de limitation angulaire par écartement de l'évidement (6) vis-à-vis du verrou (8), et
- dans la position de libération en poursuivant l'écartement de l'évidement (6) vis-à-vis du verrou (8).

4. Articulation selon la revendication 2 ou 3, **caractérisée en ce que** le moyen de réglage (3) de l'angle d'écartement de butée est réalisé sous forme de disque intermédiaire (3) avec
- une face supérieure essentiellement de forme circulaire
- une face inférieure essentiellement de forme circulaire, sensiblement parallèle à la face supérieure, et
- une surface périphérique sur laquelle est agencée la butée (7) et l'évidement (6) ou le verrou (8).

5. Articulation selon l'une des revendications 2, 3 ou 4, **caractérisée en ce que**
- quand le moyen d'arrêt (15) est dans la position de limitation angulaire, et
- quand le premier bras d'articulation et le second bras l'articulation (2) définissent l'angle d'écartement de buter (ΦA),
le verrou (8) est appliqué contre la butée (7) de sorte qu'un pivotement du premier bras d'articulation (1) par rapport au second bras d'articulation (2) au-delà de l'angle d'écartement de butée (ΦA) est bloqué.

6. Articulation selon l'une des revendications 2 à 5, **caractérisée en ce que** le verrou (8) s'engage sensiblement sans jeu dans l'évidement (6) quand le dispositif d'arrêt est dans la position d'arrêt.

7. Articulation selon l'une des revendications 2 à 6, **caractérisée en ce que**
- le disque intermédiaire (3) comporte des dents (5) au moins sur une partie de sa périphérie, et
- le dispositif d'arrêt comprend une vis tangente (4), laquelle est agencée sur le premier bras d'articulation et engrène avec les dents (5) du disque intermédiaire (3).

8. Articulation selon la revendication 7, **caractérisée en ce que** la vis tangente (4) engrène avec autoblocage avec les dents (5) du disque intermédiaire (3).

9. Articulation selon l'une des revendications 2 à 8, **caractérisée en ce que** la butée (7) est formée par une zone du disque intermédiaire (3) qui dépasse radialement depuis l'axe de pivotement (A).

10. Articulation selon l'une des revendications 2 à 9, **caractérisée en ce que**
- l'évidement (6) et
- la butée (7) se raccordent directement l'un à l'autre le long de la périphérie du disque intermédiaire (3).

11. Articulation selon l'une des revendications précédentes, **caractérisée en ce que** l'articulation comprend un second moyen d'arrêt (15), qui peut être amené dans une position de limitation angulaire dans laquelle
- le premier bras d'articulation (1) et le second bras d'articulation (2) sont capables de pivoter l'un par rapport à l'autre jusqu'à un second angle d'écartement de butée (γA) préétabli, et
- un pivotement vers des angles d'écartement plus faibles (Φ) est bloqué.

12. Orthèse comprenant
(a) un premier moyen de réception pour un premier membre corporel, et
(b) un second moyen de réception pour un second membre corporel, lesquels
- sont reliés en pivotement l'un par rapport à l'autre par au moins une articulation selon l'une des revendications précédentes, et
- définissent un angle d'orthèse (θ).

13. Orthèse selon la revendication 12, **caractérisée en ce que** le premier moyen de réception et le second moyen de réception sont reliés à au moins une seconde articulation selon l'une des revendications 1 à 11, et **en ce que**
- la première articulation, dans la position de limitation angulaire, limite le pivotement du premier moyen de réception par rapport au second moyen de réception dans une direction, et
- la seconde articulation, dans la position de limitation angulaire, limite le pivotement du premier moyen de réception par rapport au second moyen de réception dans une autre direction.

14. Orthèse selon la revendication 12 ou 13, **caractérisée en ce qu'**elle est une orthèse de genou ou une orthèse de coude.
